## Europäisches Patentamt
## European Patent Office
## Office européen des brevets

(11) Veröffentlichungsnummer: **0 155 649 B1**

(12) **EUROPÄISCHE PATENTSCHRIFT**

(45) Veröffentlichungstag der Patentschrift: **11.12.91**

(51) Int. Cl.⁵: **C07D 207/00**, C07D 213/00, C07D 241/00

(21) Anmeldenummer: **85102997.5**

(22) Anmeldetag: **15.03.85**

(54) **Verfahren zur Aromatisierung gesättigter stickstoffhaltiger Heterocyclen.**

(30) Priorität: **22.03.84 DE 3410542**

(43) Veröffentlichungstag der Anmeldung:
**25.09.85 Patentblatt 85/39**

(45) Bekanntmachung des Hinweises auf die
Patenterteilung:
**11.12.91 Patentblatt 91/50**

(84) Benannte Vertragsstaaten:
**BE CH DE FR GB LI NL**

(56) Entgegenhaltungen:
**EP-A- 0 061 982**
**EP-A- 0 099 592**
**DE-A- 1 024 080**
**DE-A- 1 192 648**
**GB-A- 1 393 086**

(73) Patentinhaber: **BASF Aktiengesellschaft**
**Carl-Bosch-Strasse 38**
**W-6700 Ludwigshafen(DE)**

(72) Erfinder: **Rebafka, Walter, Dr.**
**Lessingstrasse 4**
**W-6945 Hirschberg(DE)**

## Beschreibung

Die vorliegende Erfindung betrifft ein neues Verfahren zur Aromatisierung bestimmter gesättigter stickstoffhaltiger fünf- und sechsgliedriger Heterocyclen mittels Dehydrierung in Gegenwart eines Palladium-Katalysators, der ein Erdalkalihalogenid enthält.

Es ist bekannt, Piperidin durch Dehydrierung in Pyridin überzuführen. Nach dem Vorschlag der DE-B-1 192 648 soll dies mittels eines Trägerkatalysators mit Kieseigelträger und mit Palladium oder Platin als aktiver Komponente in Gegenwart von Wasserstoff geschehen. In diesem Verfahren ist aber die Katalysatorbelastung mit einem Wert von 0,013 1 Piperidin pro 1 Katalysator und Stunde viel zu gering. Zudem ergeben sich unbefriedigende Werte für die Katalysatorstandzeit, denn bereits nach 16 Tagen sinkt der Pyridinaustrag von 80 % auf 54 % ab.

Gemäß der GB-A-1 393 088 wird diese Umsetzung an einem Palladium/Kieselgel-Katalysator vorgenommen. Dabei können aber nur spezielle, in Essigsäure lösliche Palladiumverbindungen zur Imprägnierung des Kieselgels verwendet werden, und die anschließende Aktivierung des Katalysators gestaltet sich sehr aufwendig. Auch bei diesem Verfahren sind, wie durch die Desaktivierungsraten belegt, die Katalysatorstandzeiten schlecht.

In der EP-A-61 982 wird schließlich ein Verfahren zur Dehydrierung von unsubstituiertem oder alkylsubstituiertem Piperidin Zum jeweiligen Pyridin beschrieben. Es werden Palladium-Trägerkatalysatoren verwendet. An das Trägermaterial Kieselgel müssen dabei strenge Anforderungen bezüglich Teilchengröße und Porenvolumen gestellt werden. Zudem fällt gemäß Beispiel 1 die Produktivität von $\beta$-Picolin innerhalb von 13 Tagen auf 25 % des Ausgangswerts ab.

Auch die Dehydrierung von Pyrrolidinen zu den entsprechenden Pyrrolen ist bekannt. So wird in der DE-A-3 123 302 vorgeschlagen, die Umsetzung mit Palladium-Trägerkatalysatoren vorzunehmen, die basische Verbindungen und/oder Elemente der Gruppe 1B, 2B, 7B, Kobalt und/oder Nickel enthalten. Jedoch ergibt sich auch bei diesem Verfahren eine viel zu geringe Katalysatorbelastung (0,08 1 Pyrrolidin pro 1 Katalysator und Stunde).

In DE-A-1 024 080 wird zur Dehydrierung von Piperidin ein Platin- oder Palladium-Katalysator auf Kieselgel verwendet. Dieser Katalysator hat jedoch nur eine geringe Standzeit. GB-A-1 393 086 betrifft ein Verfahren zur Herstellung eines Palladium-auf-Kieselgel-Katalysators. Bei der Verwendung dieses Katalysators zur Dehydrierung von Piperidin zu Pyridin muß nach den Beispielen in Gegenwart von Wasserstoff gearbeitet werden. EP-A-99 592 verwendet zur Aromatisierung von Piperidin zu Pyridin ein mit Kupfer, Nickel und Chrom aktiviertes Siliciumdioxid als Katalysator.

Ein gemeinsamer Nachteil aller abgehandelter Verfahren ist weiterhin darin zu sehen, daß zur Reduktion des Katalysators, zur Aktivitätssteigerung und zum Aktivitätserhalt die Dehydrierung in Anwesenheit von zusätzlichem Wasserstoff durchgeführt wird. Dabei ergeben sich folgende Molverhältnisse:

DE-B-1 192 648 6 Mol Wasserstoff pro Mol Piperidin
GB-A-1 393 087 10 Mol Wasserstoff pro Mol Piperidin
EP-A- 61 982 3 Mol Wasserstoff pro Mol 3-Methylpiperidin
DE-A-3 123 302 2 Mol Wasserstoff pro Mol Pyrrolidin.

Diese zusätzlich (zu dem bei der Dehydrierung entstehenden Wasserstoff) verwendeten Wasserstoffmengen erfordern einen erhöhten technischen Aufwand bei der Durchführung der Verfahren. Dies zeigt sich beispielsweise in der Bereitstellung von Wasserstoff, in der Installation und Benutzung von Kreisgaspumpen, in den zusätzlichen Energiemengen, die zum Erwärmen des Wasserstoffs auf die jeweiligen Reaktionstemperatur benötigt werden oder in den zusätzlichen Maßnahmen, die bei der Kondensation des Reaktionsaustrags getroffen werden müssen.

Aufgabe der vorliegenden Erfindung war es nun, ein Verfahren bereitzustellen, das es erlaubt, stickstoffhaltige Heterocyclen zu dehydrieren, ohne daß dabei die geschilderten Nachteile in Kauf genommen werden müssen.

Dementsprechend wurde ein kontinuierliches Verfahren zur Aromatisierung gesättigter, ein oder zwei Stickstoffatome enthaltender, 5- und 6-gliedriger Heterocyclen, sowie alkylsubstituiert ein Pyrrolidin , Piperidin oder Piperazin, durch Dehydrierung in der Gasphase gefunden, das dadurch gekennzeichnet ist, daß man die Dehydrierung in Gegenwart eines Palladium-auf-Aluminiumoxid-Trägerkatalysators, der 0,1 bis 10 Gew.-% Magnesiumchlorid enthält und in Abwesenheit zusätzlichen Wasserstoffs, bei einer Temperatur von 150 bis 350 °C und bei einem Druck von 0,1 bis 10 bar durchführt.

Unter Aromatisierung soll die vollständige Dehydrierung der stickstoffhaltigen Heterocyclen verstanden werden, so daß man cyclische Systeme mit 6 $\pi$-Elektronen erhält. Entsprechend der Hückel-Regel weisen solche Systeme aromatische Eigenschaften auf.

Als gesättigte stickstoffhaltige fünf- und sechsgliedrige Heterocyclen, die im erfindungsgemäßen Verfahren als Ausgangsprodukt dienen, kommen Tetrahydroazole und -diazole sowie Hexahydroazine und -diazine in Betracht.

Als fünfgliedrige Ringe sind insbesondere zu nennen Pyrrolidin und dessen alkylsubstituierte Derivate, z.B. N-Methylpyrrolidin oder 3-Ethylpyrrolidin.

Als sechsgliedrige Ringe sind insbesondere zu nennen Piperidin und dessen alkylsubstituierte Derivate, z.B. 3-Methylpiperidin oder 4-Methylpiperidin sowie Piperazin und dessen alkylsubstituierte Derivate, z.B. 2-Methylpiperazin oder 2,3-Dimethylpiperazin.

Die Dehydrierung wird in der Gasphase in kontinuierlicher Arbeitsweise vorgenommen. Erfindungsgemäß arbeitet man dabei bei einer Temperatur von 150 bis 350° C, vorzugsweise 200 bis 300° C, und einem Druck von 0,1 bis 10 bar, vorzugsweise atmosphärischem Druck.

Das Verfahren wird im Rohrreaktor mit stückigem Katalysatormaterial oder auch im Wirbelbett durchgeführt.

Als Katalysator dient ein Palladium-Trägerkatalysator, der 0,1 bis 10 Gew.-%, vorzugsweise 1 bis 5 Gew.-%, Magnesiumchlorid jeweils bezogen auf den Katalysator, enthält.

Sein Palladiumgehalt soll 0,1 bis 10 Gew.-%, vorzugsweise 0,25 bis 5 Gew.-%, jeweils bezogen auf den Katalysator, betragen.

Das Magnesiumhalogenid kann als wäßrige Lösung gemeinsam mit den entsprechenden Palladiumsalzlösungen durch Tränken des Trägermaterials aufgebracht werden. Ein getrennter Auftrag von Palladium und Magnesiumhalogenid ist ebenfalls möglich.

Nach dem Tränkungsvorgang wird der Katalysator getrocknet.

Dies geschieht im allgemeinen bei einer Temperatur von 70 und 200° C, vorzugsweise 100 bis 140° C.

Der so erhaltene Katalysator ist bereits gebrauchsfertig und muß nicht mehr mittels Wasserstoffgas aktiviert werden, wie dies im allgemeinen bei den Verfahren des Standes der Technik erforderlich ist.

Besonders vorteilhaft ist auch die Tatsache, daß bei der Durchführung des erfindungsgemäßen Verfahrens auf die Anwesenheit von zusätzlichem Wasserstoff verzichtet werden kann.

Mittels des neuen Verfahrens lassen sich die genannten Heterocyclen bei einer Katalysatorbelastung ≧ 0,2 1 Substrat pro 1 Katalysator und Stunde in Abwesenheit von Wasserstoff als Reaktions- oder Trägergas mit hohem Umsatz dehydrieren. Die Katalysatorstandzeit ist dabei größer als 100 Tage.

Die nach dem erfindungsgemäßen Verfahren hergestellten aromatischen stickstoffhaltigen Heterocyclen sind wertvolle Zwischenprodukte, z.B. zur Herstellung von Pflanzenschutzmitteln, Arzneimitteln oder Farbstoffen.

Beispiel

In ein Quarzglasrohr mit einer Länge von 50 cm und einem Durchmesser von 2,5 cm wurden ca. 200 ml eines Katalysators eingefüllt, der 1 Gew.-% Palladium und 4,5 Gew.-% Magnesiumchlorid, jeweils bezogen auf den Katalysator, enthielt und dessen Trägersubstanz aus Aluminiumoxid bestand.

Das mit dem Katalysator gefüllte Reaktionsrohr wurde mittels einer elektrischen Außenheizung auf die jeweilige Reaktionstemperatur (vgl. Tabelle) erwärmt. Anschließend wurde das Substrat in gasförmigem Zustand kontinuierlich in das Reaktionsrohr eingebracht, wobei pro Stunde diejenige Gasmenge zugeführt wurde, die sich durch das Verdampfen von jeweils 40 ml des flüssigen Substrats ergab. Der Reaktoraustrag wurde kondensiert, und der Reaktionsumsatz und die Selektivität wurden ermittelt.

Die erhaltenen Werte sind in der Tabelle wiedergegeben.

EP 0 155 649 B1

Tabelle

| Substrat<br>Endprodukt | Temp. | Umsatz | Selektivität | Betriebsdauer |
|---|---|---|---|---|
| N-Methylpyrrolidin<br>N-Methylpyrrol | 290°C | 99,5 % | 90 % | 14 Tage |
| 3-Ethylpyrrolidin<br>3-Ethylpyrrol | 290°C | 95 % | 85 % | 14 Tage |
| Piperidin<br>Pyridin | 290°C | 99,5 % | 97 % | 20 Tage |
| 3-Methylpiperidin<br>3-Methylpyridin | 290°C | 95 % | 93 % | 100 Tage |
| 4-Methylpiperidin<br>4-Methylpyridin | 290°C | 100 % | 95 % | 4 Tage |
| 2-Methylpiperazin<br>2-Methylpyrazin | 250°C | 99 % | 95 % | 10 Tage |

Bei Abbruch der jeweiligen Versuche besaß der Katalysator noch seine volle Aktivität.

**Patentansprüche**

1. Kontinuierliches Verfahren zur Aromatisierung gesättigter, ein oder zwei Stickstoffatome enthaltender, 5- und 6-gliedriger Heterocyclen, sowie alkylsubstituiertem Pyrrolidin, Piperidin oder Piperazin, durch Dehydrierung in der Gasphase, dadurch gekennzeichnet, daß man die Dehydrierung in Gegenwart eines Palladium-auf-Aluminiumoxid-Tragerkatalysators,der 0,1 bis 10 Gew.-% Magnesiumchlorid enthält und in Abwesenheit zusätzlichen Wasserstoffs, bei einer Temperatur von 150 bis 350° C und bei einem Druck von 0,1 bis 10 bar durchführt.

2. Verfahren nach Anspruch 1, dadurch gekennzeichnet, daß man die Aromatisierung bei einer Temperatur von 200 bis 300° C durchführt.

**Claims**

1. A continuous process for the aromatization of a saturated five-membered or six-membered heterocycle containing one or two nitrogen atoms and alkyl-substituted pyrrolidine, piperidine or piperazine by dehydrogenation in the gas phase, wherein the dehydrogenation is carried out in the presence of a supported palladium-on-alumina catalyst containing from 0.1 to 10% by weight of magnesium chloride and in the absence of additional hydrogen, at from 150 to 350° C and under from 0.1 to 10 bar.

2. A process as claimed in claim 1, wherein the aromatization is carried out at from 200 to 300° C.

**Revendications**

1. Procédé en continu d'aromatisation d'hétérocycles penta- et hexagonaux saturés contenant un ou deux atomes d'azote, ainsi que de pyrrolidine, de pipéridine ou de pipérazine alkylsubstituée, par déshydro-

4

génation en phase gazeuse, caractérisé en ce qu'on conduit la déshydrogénation en présence d'un catalyseur de palladium fixé sur un support d'oxyde d'aluminium qui contient de 0,1 à 10% en poids de chlorure de magnésium et en l'absence d'hydrogène supplémentaire, à une température de 150 à 350° C et sous une pression de 0,1 à 10 bar.

2. Procédé selon la revendication 1, caractérisé en ce qu'on conduit l'aromatisation à une température de 200 à 300° C.